# EUROPEAN PATENT SPECIFICATION

(11) **EP 1 699 441 B1**
(45) Date of publication and mention of the grant of the patent: **19.05.2010**
(21) Application number: 04804872.2
(22) Date of filing: 16.12.2004
(51) Int. Cl.: A61K 9/20

(54) **METHOD OF MANUFACTURE OF FAST-DISINTEGRATING TABLETS**
VERFAHREN ZUR HERSTELLUNG VON SCHNELL AUFLÖSENDEN TABLETTEN
PROCEDE DE FABRICATION DE COMPRIMES A DELITEMENT RAPIDE

(30) Priority: 17.12.2003 EP 03405901
(43) Date of publication of application: 13.09.2006
(73) Proprietor: Pantec AG, 4313 Möhlin (CH)
(72) Inventor: BAUER, Kurt, Heinz, 79112 Freiburg (DE); ROHRER, Hans-Peter, CH-4313 Möhlin (CH)
(74) Representative: Becker, Konrad
(86) International application number: PCT/EP2004/053525
(87) International publication number: WO 2005/058284

(56) References cited:
- WO-A-02/062152
- US-A- 4 371 516
- US-A- 5 976 577

## Description

### Field of the Invention

The invention relates to a method of manufacture of fast-disintegrating tablets, and tablets obtained comprising chemicals, foodstuff, oral drug components and the like.

### Background Art

The art of tablet making involves the making of a composition containing an active ingredient which is sturdy for packaging and handling, and disintegrable in a predictable manner.

Fast-dissolving and fast-disintegrating tablets are especially important in the field of orally ingested drugs. Many people are unwilling and/or unable to swallow tablets, capsules or other traditional solid dosage forms. This is especially the case of pharmaceuticals for paediatric or geriatric use.

One approach suitable for these persons is the use of effervescent tablets or granules. However, the use of effervescent tablets requires preparatory steps before administration of the drug and the presence of water and a suitable mixing container. In addition, the manufacture and stability of effervescent tablets is often problematic. Another possibility is the use of a chewing gum or chewing tablet containing a drug capable of absorption through the buccal cavity (U.S. Pat. No. 5,225,197). Substantial disadvantages inherent in such a delivery system are that many active drug ingredients are not suitable for buccal absorption and that many persons are not able to chew gums or tablets because of braces, dental work, and the like. Furthermore, gums are often difficult to prepare.

Two main technologies are presently used to obtain pharmaceutical dosage forms for fast disintegration on contact with saliva in the buccal cavity:
(1) The active ingredient is mixed with water-soluble diluents and compressed on a tableting machine at low to medium compression force. This is the more conventional approach, and very often does not give tablets with the required tensile strength and reasonable disintegration time. A more recent approach is the OraSolv™ technology, which involves incorporating microencapsulated drug ingredients into a tablet obtained by compression (U.S. Pat. No. 5,178,878). The tablets have to be packed into special peel-off blister packs because their mechanical resistance is insufficient in normal blister packs. Rapidly dissolving tablets have been produced using suitable crystalline sugar structures under adapted curing conditions (U.S. Pat. No. 5,866,163). Further compressed, rapidly dissolvable dosage forms including an active ingredient and a matrix composed of a nondirect compression filler and a lubricant are disclosed in U.S. Pat. No. 6,221,392.
(2) A suspension is prepared with the active ingredient and appropriate excipients. The suspension is dispensed into blister packs and freeze-dried (U.S. Pat. No. 4,371,516). This approach usually gives tablets with porous structure, reasonable tensile strength and disintegration time, but is time-consuming and requires a costly freeze-drying process. Furthermore, the effectiveness of a freeze-drying process always depends on the physico-chemical parameters of the active substances used, and is not suitable for substances having a limited solubility in water. Replacing the freeze-drying step by conventional drying at room temperature or elevated temperature, also drying with microwave radiation, is disclosed in International Patent Application WO 97/38679, but is likewise time-consuming, and is also limited to active substances which survive such conditions.

Fast-disintegrating tablets are also applicable for convenient portioning of dehydrated foodstuffs, e.g. for coffee, milk, cocoa, tea, gravy, soup and other drinks, and are used with cold or hot water to reconstitute the original liquid foodstuff. In contrast to tablets used, as orally digestible pharmaceuticals or vitamins and the like, tablets to be dissolved in water to reconstitute an original aqueous preparation are not supposed to contain additives influencing the appearance or organoleptic properties of the original foodstuff. In particular lubricants used to facilitate the separation of tablets from moulds and from the dies and punches which ram the powders into the moulds are not desirable, since in general, such lubricants are insoluble in water and will leave a residue after tablet disintegration, at a cost of consumer appeal in a transparent product such as coffee, tea and clear soups.

Fast-disintegrating tablets to be dissolved in water or also in use in other fields, such as fabric (laundry) washing or dishwashing, as bleaching tablets, sanitization tablets, water treatment tablets, denture cleansing tablets, and also for cleaning apparel and removing calcifications. "Active ingredient" under these circumstances are detergents, acids or other chemicals. Tablets ease the portioning of these compounds and are much easier to handle than powders, granules or viscous or non-viscous solutions and suspensions.

Detergent compositions in tablet form are easier to handle and dispense into the wash load, and they are more compact, hence facilitating more economical storage. Such tablets are generally made by compressing or compacting a quantity of detergent composition in particulate form. It is desirable that tablets should have adequate mechanical strength when dry, yet disintegrate and disperse and dissolve quickly when added to wash water. U.S. Pat. No. 3,081,267 taught that the force, and hence pressure, applied when compacting a composition into tablets should be limited, or else the tablets would take too long to dissolve. Some documents have proposed surface treatments or coatings to enhance tablet strength. U.S. Pat. No. 3,451,928 states that the problem of strength versus speed of dissolution remained unsolved, and proposed a treatment of spraying on water, followed by flash heating. It is further known to include materials whose function is to enhance disintegration of tablets when placed in wash water, e.g. urea or sodium citrate.

### Summary of the Invention

The invention relates to a method of manufacture of fast-disintegrating tablets, **characterized in that** the components in pulverized form are contacted with a pressurized liquefied gas or gas mixture, homogenized, introduced into moulds under pressure, and decompressed. The pressurized liquefied gas or gas mixture may further comprise a low-boiling solvent. In this way tablets or tablet-like solid dosage forms are obtained with a similar porous structure as usually result from freeze-drying processes.

### Detailed Description of the Invention

Fast-disintegrating tablets are important in a number of different fields of application. For example they may be used for textile washing or in dishwashers, and comprise detergents and suitable additives. Other applications are e.g. as bleaching tablets, sanitization tablets, water treatment tablets, denture cleansing tablets, and the like. For decalcification of apparatus running with hot water, e.g. coffee machines, hot water pots, and nozzles in showers and the like, such tablets usually contain acidic chemicals able to dissolve calcifications. More generally tablets of chemicals may be used in many different applications where convenient portioning of chemicals is desirable, and the resulting portion preferably should disintegrate in water or aqueous solvent within a reasonable period of time.

A particular application of fast-disintegrating tablets is in the field of foodstuffs, e.g. for coffee, tea, cocoa or powdered m ilk, gravy, soup or other drinks, where the tablet is to be dissolved in cold or hot water to reconstitute the original foodstuff, or in tablets of edible energy source to be taken directly into the mouth without water, e.g. fast energy providers to be eaten and digested during periods of continuous activity such as running or biking and similar sports.

Another particular application is for medicaments for oral consumption, e.g. drugs taken orally which do not have to be swallowed in one portion, but supposed to rapidly disintegrate in contact with saliva in the buccal cavity. Such fast-disintegrating tablets are particularly important as pharmaceutical formulations in paediatric and geriatric uses, since children and elderly people sometimes have difficulty in swallowing tablets or capsules, and disintegrated medication is much easier to swallow as a soft and pasty mass. Another particular application for fast-disintegrating tablets is for medication to be taken independent of availability of water or other liquids to aid swallowing. Fast-disintegrating tablets are also useful for veterinary use.

The expression "tablets" as used herein is not limited to a particular size or form of compacted material. Tablets may have many different appearances, such as classical dish-like shapes, but also other spherical or ellipsoid shapes, rods, granules, blocks, cubes with rounded edges, or particular forms as obtainable from a suitable mould. Size may vary from approximately 1.5 mm diameter or 1.5 mm extension in the longest direction, so-called micro-tablets or pellets, to an approximate size of a golf ball or the like. Tablets for direct oral consumption are of course more limited in size, e.g. of a size between approximately 1.5 mm to approximately 30 mm, preferably in the range of 2 to 10 mm.

The method of manufacture of the invention is related to the classical method of wet granulation, but only in the preliminary stages. Starting point is a pulverized mixture comprising a suitable binding agent. For aggregation such pulverized mixtures used to be moistened with a suitable solvent, for example water or aqueous solvents, or sometimes also organic solvents, such as alcohols. For the preparation of sintered tablets a pulverized mixture containing a binding agent on contact with water or the corresponding solvent starts to aggregate into granules or larger aggregates.

In contrast to these methods the present invention uses liquefied or compressed gases or gas mixtures under high pressure, optionally in the presence of low-boiling solvents to moisten the dry pulverized mixture. If mixtures of gases are used, these preferably should be azeotropic mixtures. The pressurized liquefied gas has a boiling point below 20° Celsius, preferably below 0° Celsius, at normal pressure (101325 Pa, 1.01325 bar). Gases considered are chemically inert gases, primarily those to be used in pressurized aerosols. Examples of such gases are fluoroalkanes or fluorochloroalkanes, for example TG 11 (trichlorofluoromethane), TG 12 (dichlorodifluoromethane), TG 114 (1,2-dichloro-1,1,2,2-tetrafluoroethane), TG 227 (1,1,1,2,3,3,3-heptafluoropropane), TG 125 (pentafluoroethane), TG 134a (1,1,1,2-tetrafluoroethane) and TG 152a (1,1 -difluoroethane), hydrocarbons such as lower alkanes, for example propane, n-butane or isobutane, or a gaseous ether, for example dimethyl ether. Preferred are the fluoroalkanes TG 227 and 134a, the lower alkanes propane, n-butane or isobutane, and dimethyl ether.

Low-boiling solvents have boiling points from 20° Celsius up to below 100° Celsius, preferably between 40° and 85° Celsius, at 101325 Pa (normal pressure). Examples of such solvents are methanol, ethanol, isopropanol, acetone, ethyl acetate and methylene chloride.

Pressure required for handling these gases, and for the corresponding moistened and plasticized masses to be transferred into moulds, are between normal pressure (101325 Pa) and up to 100 bar (10⁷ Pa), preferably between normal pressure and 20 bar (2 x 10⁶ Pa). The preferred gases are those which can be liquefied at or around room temperature, e.g. at 20° Celsius, with a pressure of up to 10 bar (10⁶ Pa).

Suitable containers and apparatus have to withstand theses pressures, and are in particular autoclaves, optionally with corresponding stirrers to dissolve, homogenize and/or produce a mouldable plasticized mass, and connected through suitable locks and valves, means to control pressure and means to transfer from one container to another container. The final container has to have proper moulds to form the tablets of predetermined size and form, and means to control pressure, preferably such as to continuously form tablets under pressure and then eject them after decompression.

Since the usual binding agents tend to be insoluble or sparingly soluble in lower alkanes, fluoroalkanes or fluorochloroalkanes, it is sometimes advisable to use mixtures of such gases together with low-boiling solvents, in particular with acetone, ethyl acetate, ethanol or isopropanol, preferably ethanol, isopropanol and acetone. Azeotropic mixtures are particularly preferred. Alternatively or additionally, carbon dioxide may be added to the gas or gas mixture to help controlling solubility of a binding agent or other components in the corresponding pressurized liquefied gases, gas mixtures, or mixture of gases and low-boiling solvents. Carbon dioxide may be added up to a pressure of 50 - 80 bar (5 - 8 x 10⁶ Pa), and this is especially useful in the case of the presence of low-boiling solvents, e.g. in the presence of acetone, ethyl acetate, ethanol, isopropanol or n-propanol.

The pulverized mixture may have uniform size or different classes of particle sizes, depending on the physico-chemical properties of the active ingredient(s) and the fillers and other components, the intended use, and the liquefied or compressed gases and optional low-boiling solvent to be used. Depending on the solubility of the active ingredient (and the bioavailability of a drug substance in the case of pharmaceuticals) the particle size may be e.g. between approximately 0.1 □m and 1 mm, in particular between 1 □m and 0.2 mm. The particle size of the excipients may vary between 20 µm and 1 mm, particularly between 50 µm and 300 µm. The mixture may also contain larger aggregates readily dissolvable in the liquefied or compressed gases and optional low-boiling solvents.

The amount of the liquefied or compressed gases is chosen such as to moisten the pulverized mixture in order to get a homogenous plasticized mass, highly viscous suspension or sticky mass easily mouldable. When using low-boiling solvents it may also suffice to moisten the pulverized mixture on the outer surface in order to make them partially sticky.

The homogenized pulverized mixture containing the active ingredient(s), fillers able to build up porous structures and other components is introduced in a container or apparatus suitable for handling high pressure, or otherwise components of such a mixture are introduced and then intimately mixed in the corresponding container or apparatus. The proper amount of the liquefied or compressed gas or gas mixture and optional low-boiling solvent is then added under pressure, and the obtained plasticized mass pre-formed under gas pressure in corresponding particularly constructed moulds. Such moulds may at the same time be constructed in a way to function as pressurizable locks and valves. The moulded mass is then transported to an area of normal pressure or slightly reduced pressure. In this area, liquefied gases are decompressed and the low-boiling solvents evaporated within a very short time period. Care must be taken to compensate the temperature fall caused by decompression and evaporation. If solvents have been added which have a boiling point above room temperature, short heating with an infrared lamp or short treatment with microwaves may be required to eliminate these solvents.

As is readily understandable from the description of the method of the invention, this procedure is less time-consuming and less energy-consuming than a comparable method using water or aqueous or organic solvents wherein the excess solvent has to be evaporated by heat or freeze-drying or other energy source. On the other hand, tablets are obtained with a similar porous structure as usually result from freeze-drying processes.

The apparatus or container to be used in the method of the invention may be constructed such as to allow recycling of the gases and low-boiling solvent used in forming the tablets. The tablets when entering the normal or low-pressure compartment are easily collected and ejected from the mould without the need for proper lubricants. Tablets so produced may immediately be packed into suitable containers for transport and use, for example regular or peel-of blister packs, depending on the property of the tablet components, their tensile strength, and the intended use.

In a particular embodiment of the method the active ingredient alone or the active ingredient containing some of the additional components, in pulverized form, may be introduced into the apparatus or container, and the other component(s) of the final tablet pre-dissolved in the liquefied or compressed gas or gas mixture and optional low-boiling solvent, homogenized and then added to the active ingredient. Such other ingredients may be binding agent(s), solubilizing additive(s), softener(s) and the like.

The obtainable tablets have a highly porous structure due to the efficient, short-time decompression and evaporation of the gas or solvent used in the compacting step.

Whereas apparatus for tableting pulverized mixture with conventional methods usually require high-grade steel to stand the pressures involved in compacting, the present methods also allow the use of other materials for the construction of the moulds, e.g. softer and deformable type of materials such as polymers, e.g. teflon, polyamides, and the like, and be particularly adapted to provide a desired form to the tablet, support sealing of the apparatus or locks and valves in the manufacturing process, and simplify separation of the tablets after decompression.

Pharmaceutical dosage forms for oral administration prepared according to the present invention may comprise one or more active ingredients, fillers, binding agents, and further auxiliaries.

Active ingredients are especially pharmaceuticals but may be also, for example, vitamins, minerals or dietary supplements. Pharmaceuticals may include, without limitation, antacids, analgesics, anti-inflammatory agents, antibiotics, laxatives, anorexics, antiasthmatics, diuretics, anti-flatulents, anti-migraine agents, anti-arrhythmic agents, antispasmodics, sedatives, anti-hyperactive agents, tranquilizers, antihistamines, decongestants, beta-blockers, coronary vasodilators, bronchodilators, muscle relaxants, anticoagulants, anti-leptic agents, anti-emetics, hypotensives, sympathomimetic agents, expectorants, oral anti-diabetic agents, hormones and combinations thereof.

The filler can be chosen from those known in the art including sugars, sugar alcohols, cellulose, and calcium phosphates and sulfates, and are, for example, mannitol, lactose, dicalcium phosphate, calcium sulphate, sucrose, saccharose, glucose, fructose, sorbitol and xylitol, in particular mannitol. Fillers are able to build up porous structures, support rapid disintegration and preferably have a non-hygroscopic character. The filler is usually present in an amount of between 50 and 99 % (weight) of the total dosage form.

The binding agent is primarily used to give sufficient consistency to the formulation to avoid breaking of the article when removed from blisters and during handling. Usual binding agents are those applied in classical tableting, e.g. polyethylene glycols, acacia, tragacanth, starch, cellulose materials such as ethyl cellulose, methyl cellulose, hydroxypropyl cellulose, hydroxypropyl methyl cellulose (HPMC), cellulose acetate propionate, HPMC acetate phthalate, HPMC acetate succinate, and the like; methacrylate polymers and methacrylic acid acrylate copolymers such as known under the trade names Eudragit^{™} and Kollicoat^{™}, and the like; shellac, polyvinyl acetate phthalate, polyvinyl pyrrolidones, polyvinyl pyrrolidone-polyvinyl acetate copolymers, alginic acid or a salt or an ester thereof, carrageenan gum, xanthan gum, gellan gum, and gelatin.

Binding agents to be used have to be soluble or partially soluble in the liquefied gases or optional low-boiling solvent, and have to be chosen accordingly. For the preferred hydrophobic gases of the invention the following binders are particularly preferred: hydroxypropylcellulose phthalate or succinate, ethylcellulose, cellulose phthalate, polyvinyl phthalate, and methacrylic acid acrylate copolymers, in particular those available under the trade names EUDRAGIT^{™} E, L or S.

Further auxiliaries are, for example, lubricants, such as talc, magnesium stearate or calcium stearate, stearic acid, polyethylene glycols, sodium stearyl fumarate, hydrogenated vegetable oil, leucine or iso-leucine, or a behenic acid derivative and the like. Since these are primarily used to avoid sticking of the tableted product to the surface of a mould, their use can be avoided in the present invention. Other auxiliaries (adjuvants) known in the art include disintegrants, such as starches, super disintegrants, such as sodium carboxymethylstarch, insoluble sodium carboxymethyl cellulose, and cross-linked polyvinylpyrrolidone, and effervescents, softeners (and hydrophilization agents), such as glycerol, propylene glycol, poloxamer or Pluronics^{™}, i.e. copolymers of polyethylene glycol and polypropylene glycol, flavours, aromas, sweeteners, colorants, buffering agents, acidifying agents, diluents, preservatives and the like.

The active ingredient is normally used as the pure substance in different crystalline forms but it can also be coated or micro- or nano-encapsulated.

As a result of the particular process of manufacture used, the dosage form of the invention normally has a density of 200-1000 mg/ml, preferably 300-900 mg/ml, more preferably 600-900 mg/ml, or 400-800 mg/ml. This is a density that is much lower than that of compressed dosage forms like normal tablets (having densities of above 1000 mg/ml). As a result of its unusually low density, the dosage form of the invention disintegrates more rapidly than would be the case, if the mixture of its components are subjected to compression force.

The finished tablet units may e.g. be sealed in blister packs, special peel-of blister packs for tablets of low tensile strength, or other containers for storage.

The dosage form is presented e.g. as a tablet of a size and shape adapted for direct oral administration to a patient. The tablet is pleasant to take and, once placed into the mouth, will disintegrate substantially and instantly without any voluntary action by the patient, e.g. chewing. Upon disintegration of the tablet, the active ingredient is released and can be swallowed as a suspension or absorbed from the buccal cavity. The size of tablets for foodstuff or technical applications may be much bigger than that of pharmaceutical products.

The dosage form according to the present invention is convenient to use for the consumer without the need of water or additional devices. Moreover, the instant disintegration and/or dissolution gives a sensation of a rapid and powerful action of the pharmaceutical dosage form and makes it unique and motivating for the patient to take.

Since this method of the invention combines directly the mixing, kneading and moulding steps of the tableting process and dispenses of a complicating and expensive additional drying step, it is more economical than prior art methods using other aqueous or nonaqueous solvents for the tableting process, albeit providing similar porous, fast-disintegrating and fast-dissolving tablets with desired properties as are available with those more conventional processes. An additional economic aspect is also the ease of potential recycling of gases and low-boiling solvent used in the process.

The following examples illustrate the invention, but in now way limit the scope thereof.

### Example 1: Regular fast-disintegrating tablets, diclofenac

In a autoclave with stirrer diclofenac sodium (2.5 kg), mannitol (20.0 kg), sodium carboxymethylcellulose (of a medium viscosity, 1.0 kg), aspartam (1.0 kg), and orange flavour (0.3 kg) are mixed to homogeneity. In a separate autoclave, Eudragit^{™} E 100 (dimethylaminoethyl methyl methacrylate copolymer, 1.5 kg) is dissolved in a minimum amount of dimethyl ether (DME, ca. 10 kg) to get a solution. This solution is transferred through a pressure valve to the stirred mixture in the first autoclave. Stirring is continued until a moist formable mass is obtained. Depending on the particle size of the components additional dimethyl ether has to be added. Carbon dioxide (3.0 kg) is added through a pressurized valve, and the moist plasticized mass is transferred into a mould apparatus for exactly measured tablets under pressure. The moulds are decompressed, and the obtained solidified tablets ejected. The final weight of the dry tablets is 230 mg. They are sealed into blister packs in a standard packaging machine.

### Example 2: Coated and stabilized active ingredient, acetylsalicylic acid

Acetylsalicylic acid (3.0 kg) is stabilized by mixing with a concentrated solution of anhydrous citric acid (0.3 kg) in ethanol, and evaporated to dryness. The stabilized acetylsalicylic acid crystals are then coated with aqueous ethanolic ethylcellulose (0.1 kg) according to standard procedures in a fluidized bed reactor, and dried. In a stirred autoclave the coated acetyl salicylic acid, anhydrous monosodium citrate (3.5 kg), sodium hydrogen carbonate (1.6 kg), sodium carbonate (0.2 kg), mannitol (2.5 kg), sodium carboxymethyl starch (Na-CMS, 0.5 kg), saccharin sodium (sweetener, 0.05 kg), and raspberry flavour (0.25 kg) are intimately mixed to homogeneity. In a separate autoclave, Eudragit^{™} L 100 (methacrylic acid methyl methacrylate 1:1 copolymer, 0.5 kg) is dissolved in TG 227 (1,1,1,2,3,3,3-heptafluoropropane, 2.0 kg) and dimethyl ether (DME, 2.5 kg) to get a solution. This solution is transferred through a pressure valve to the stirred mixture in the first autoclave. Stirring is continued until a moist formable mass is obtained. Depending on the particle size of the components additional TG 227 and DME has to be added. The moist plasticized mass is transferred into a mould apparatus for exactly measured tablets under pressure. The moulds are decompressed, and the obtained solidified tablets ejected. The final weight of the dry tablets is 1250 mg. They are sealed into blister packs in a standard packaging machine.

### Example 3: Fabric detergent

Sodium carbonate (10.0 kg), Myrj^{™} 53 (polyethylene glycol 50 stearate, non-ionic surfactant, 10.0 kg), sodium lauryl sulfate (anionic tenside, 5.0 kg), Britesil^{™} (zeolith A, amorphous sodium disilicate, 30.0 kg), and Hysorb^{™} (super absorbant, 3.0 kg) are mixed in a autoclave with a stirrer to homogeneity. In a separate autoclave, stearic acid (1.0 kg) is dissolved in a mixture of iso-butane (1.0 kg), propane (1.0 kg) and dimethyl ether (2.0 kg) under pressure. This solution is transferred through a pressure valve to the stirred mixture in the first autoclave. Stirring is continued until a moist formable mass is obtained. Depending on the particle size of the components additional dimethyl ether has to be added. Carbon dioxide is added until saturation through a pressurized valve, and the moist mass is transferred into a mould apparatus for portioning under pressure. The moulds are decompressed, and the obtained solidified portions of about 50 g ejected. They are packaged in suitable containers.

### Example 4: Decalcification agent

Sodium hexametaphosphate (990 g), tetrasodium pyrophosphate (6.0 g), sodium metaphosphate (3.5 g) and polyacrylic acid (0.5 g) are mixed in a autoclave with a stirrer to homogeneity. In a separate autoclave, acetone (30-40 g), isopropanol (30-40 g) and dimethyl ether (70 g) are mixed, and carbon dioxide (30 g) is added under pressure. This solvent mixture is transferred through a pressure valve to the stirred mixture in the first autoclave. Stirring is continued until a moist formable mass is obtained. Depending on the particle size of the components additional dimethyl ether has to be added. The ratio of acetone to iso-propanol may be varied to influence the partial solubility of the solid components and the deposition and sintering of solids. Further carbon dioxide is added in order to limit dissolution of the solid components. The moist plasticized mass is transferred into a mould apparatus for portioning under pressure. The moulds are decompressed, and the obtained solidified portions dried during approximately 10 seconds under an IR lamp, and ejected. The tablets weighing about 30 g are packaged in suitable containers.

### Example 5: Peppermint essence

Standardized peppermint extract (0.5 kg), saccharose (2.0 kg) and mannitol (0.5 kg) are mixed in an autoclave with a stirrer to homogeneity. In a separate autoclave, ethanol (30 g), dimethyl ether (150 g) and TG 227 (70 g) are mixed under pressure, and carbon dioxide (50 g) is added. This solution is transferred through a pressure valve to the stirred mixture in the first autoclave. Stirring is continued until a moist formable mass is obtained. Depending on the particle size of the components additional dimethyl ether has to be added. The moist plasticized mass is transferred into a mould apparatus under pressure. The moulds are decompressed, and the obtained solidified portions dried during approximately 10 seconds under an IR lamp, and ejected. The final weight of the dry tablets is 3 g. These are packaged in suitable containers.

### Example 6: Alpine herbs essence

Alpine herbs dry extract (1.0 kg), Palatinit^{™} (reduced palatinose, isomalt, 0.95 kg) and dextrin (0.05 kg) are mixed in an autoclave with a stirrer to homogeneity. In a separate autoclave, dimethyl ether (100 g), TG 134a (70 g) and TG 227 (40 g) are mixed under pressure, and carbon dioxide (50 g) is added. This mixture of liquefied gases is transferred through a pressure valve to the stirred mixture in the first autoclave. Stirring is continued until a moist formable mass is obtained. Depending on the particle size and solubility of the herb extract additional dimethyl ether (which increases solubility) or carbon dioxide (which reduces solubility) has to be added. The moist plasticized mass is transferred into a mould apparatus under pressure. The moulds are decompressed, and the obtained solidified portions weighing approx. 2 g ejected with a die, and packaged in a suitable container.

### Example 7: Nano- or micro-particle tablets

Drug nano- or micro-particles (1.5 kg), prepared by known methods from a nano- or micro-emulsion of a drug or embedding a drug into lipids, and mannitol (0.95 kg) are mixed in an autoclave with a stirrer to homogeneity. In a separate autoclave, ethyl cellulose (50 g) is dissolved in dimethyl ether (100 g), then added under pressure trough a suitable valve to the drug/mannitol mixture. TG 134a (50 g) and TG 227 (50 g) are added through the same valve, and the mixture stirred until a moist formable mass is obtained. Depending on the properties of the nano- or micro-particles the addition of more gases is required. Carbon dioxide (50 g) is added to saturation, and the moist plasticized mass transferred into a mould apparatus for exactly measured tablets under pressure. The moulds are decompressed, and the obtained solidified tablets ejected with a die, and packaged in a suitable container.

### Example 8: COX-2 inhibitor

Valdecoxib (COX-2 inhibitor, 1.0 kg) and Palatinit^{™} (reduced palatinose, isomalt, 2.95 kg) are mixed in an autoclave with a stirrer to homogeneity. In a separate autoclave, Eudragit^{™} L 100 (methacrylic acid methyl methacrylate 1:1 copolymer, 50 g) is dissolved in dimethyl ether (100 g), and TG 134a (100 g) and TG 227 (100 g) are added. This mixture is transferred through a pressure valve to the stirred mixture in the first autoclave. Stirring is continued until a moist formable mass is obtained. Finally, carbon dioxide (100 g) is added, and the moist plasticized mass is transferred into a mould apparatus for exactly measured tablets under pressure. The moulds are decompressed, and the obtained solidified tablets weighing 40 mg (and containing 10 mg active ingredient) ejected with a die, and packaged in a suitable container.

### Example 9: Neuroamidase inhibitor

Oseltamvir (neuramidase inhibitor, prophylactic and therapeutic agent against influenza A, 7.5 kg) and mannitol (12.5 kg) are mixed in an autoclave with a stirrer to homogeneity. In a separate autoclave, Eudragit^{™} L 100 (100 g) is dissolved in dimethyl ether (800 g) and TG 134a (700 g). This mixture is transferred through a pressure valve to the stirred mixture in the first autoclave. Stirring is continued until a moist formable mass is obtained. Finally, carbon dioxide (500 g) is added, and the moist plasticized mass is transferred into a mould apparatus for exactly measured tablets under pressure. The moulds are decompressed, and the obtained solidified tablets weighing 200 mg (and containing 75 mg active ingredient) ejected with a die, and packaged in a suitable container.

### Example 10: Cholesterol resorption inhibitor

Ezetimib (cholesterol resorption inhibitor, 10.0 kg), saccharose (1.25 kg) and lactose (37.75 kg) are mixed in an autoclave with a stirrer to homogeneity. In a separate autoclave, Kollidon^{™} VA 64 (polyvinylpyrrolidone/polyvinyl acetate 60:40, 1.0 kg) is dissolved in ethanol (1.0 kg) and dimethyl ether (1.5 kg), and TG 134a (1.2 kg) is added. This mixture is transferred through a pressure valve to the stirred mixture in the first autoclave. Stirring is continued until a moist formable mass is obtained. Finally, carbon dioxide (1.5 kg) is added, and the moist plasticized mass is transferred into a mould apparatus for exactly measured tablets under pressure. The moulds are decompressed, the solidified tablet dried by a short treatment under an IR lamp, the obtained tablets weighing 50 mg (and containing 10 mg active ingredient) ejected with a die, and packaged in a suitable container.

## Claims

1. A method of manufacture of fast-disintegrating tablets, **characterized in that** the components in pulverized form are contacted with a pressurized liquefied gas or gas mixture, homogenized, introduced into moulds under a pressure of between 101325 Pa (normal pressure or 1.01325 bar) and 10⁷ Pa (100 bar), and decompressed.

2. The method of claim 1 wherein the pressurized liquefied gas has a boiling point below 0° Celsius at 101325 Pa (normal pressure).

3. The method of claim 1 or 2 wherein the pressurized liquefied gas is selected from fluoroalkanes, fluorochloroalkanes, lower alkanes and low-boiling ethers, and mixtures thereof.

4. The method of claim 3 wherein the gas mixture is an azeotropic mixture.

5. The method of any one of claims 1 to 4 wherein the pressurized liquefied gas is selected from TG 227 (1,1,1,2,3,3,3-heptafluoropropane), TG 134a (1,1,1,2-tetrafluoroethane), propane, n-butane, isobutane and dimethyl ether, and mixtures thereof.

6. The method of any one of claims 1 to 5 wherein the pressurized liquefied gas or gas mixture further comprises a low-boiling solvent having a boiling point between 20° and 100° Celsius at 101325 Pa (normal pressure).

7. The method of claim 6 wherein the liquefied gas or gas mixture and the further solvent form an azeotropic mixture.

8. The method of any one of claims 1 to 7 wherein the pressurized liquefied gas further comprises carbon dioxide.

9. The method of any one of claims 1 to 8 wherein the pressurized liquefied gas or gas mixture and optional low-boiling solvent is first used to dissolve a binding agent, and the resulting solution added to the other solid components in pulverized form under pressure.

10. The method of any one of claims 1 to 9 wherein the components comprise a binding agent selected from hydroxypropylcellulose phthalate and succinate, ethylcellulose, cellulose phthalate, polyvinyl phthalate, and methacrylic acid acrylate copolymers.

11. The method of any one of claims 1 to 10 for the manufacture of pharmaceutical tablets for oral use.

12. The method of claim 11 wherein the components comprise a filler selected from sugars, sugar alcohols, cellulose, and calcium phosphates and sulfates.

13. The method of any one of claims 1 to 10 for the manufacture of tablets comprising foodstuffs or chemicals for disintegration in water or aqueous solvents.

## Patentansprüche

1. Verfahren zur Herstellung schnell zerfallender Tabletten, **dadurch gekennzeichnet, dass** die Bestandteile in pulverisierter Form mit einem unter Druck verflüssigten Gas oder Gasgemisch in Kontakt gebracht, homogenisiert, bei einem Druck von zwischen 101325 Pa (Normaldruck oder 1.01325 bar) und 10⁷ Pa (100 bar) in Schablonen eingebracht und dekomprimiert werden.

2. Verfahren gemäss Anspruch 1, worin das unter Druck verflüssigte Gas einen Siedepunkt unter 0° Celsius bei 101325 Pa (Normaldruck) hat

3. Verfahren gemäss Anspruch 1 oder 2, worin das unter Druck verflüssigte Gas aus Fluoralkanen, Fluorchloralkanen, Niedrig-Alkanen und niedrig siedenden Ethern und Mischungen davon ausgewählt wird.

4. Verfahren gemäss Anspruch 3, worin das Gasgemisch eine azeotrope Mischung ist.

5. Verfahren gemäss irgendeinem der Ansprüche 1 bis 4, worin das unter Druck verflüssigte Gas aus TG 227 (1,1,1,2,3,3,3-Heptafluorpropan), TG 134a (1,1,1,2-Tetrafluorethan), Propan, n-Butan, Isobutan und Dimethylether und Mischungen davon ausgewählt wird.

6. Verfahren gemäss irgendeinem der Ansprüche 1 bis 5, worin das unter Druck verflüssigte Gas oder Gasgemisch weiter ein niedrig siedendes Lösungsmittel mit einem Siedepunkt zwischen 20° und 100° Celsius bei 101325 Pa (Normaldruck) umfasst.

7. Verfahren gemäss Anspruch 6, worin das verflüssigte Gas oder Gasgemisch und das weitere Lösungsmittel eine azeotrope Mischung bilden.

8. Verfahren gemäss irgendeinem der Ansprüche 1 bis 7, worin das unter Druck verflüssigte Gas weiter Kohlenstoffdioxid umfasst.

9. Verfahren gemäss irgendeinem der Ansprüche 1 bis 8, worin das unter Druck verflüssigte Gas oder Gasgemisch und das allfällige niedrig siedende Lösungsmittel zuerst verwendet werden, um ein Bindemittel zu lösen, und die entstandene Lösung unter Druck zu den anderen festen Bestandteilen in pulverisierter Form zugegeben wird.

10. Verfahren gemäss irgendeinem der Ansprüche 1 bis 9, worin die Bestandteile ein Bindemittel ausgewählt aus Hydroxypropylcellulosephthalat und -succinat, Ethylcellulose, Cellulosephthalat, Polyvinylphthalat und Methacrylsäure-Acrylat-Kopolymere umfassen.

11. Verfahren gemäss irgendeinem der Ansprüche 1 bis 10 für die Herstellung von pharmazeutischen Tabletten für orale Verwendung.

12. Verfahren gemäss Anspruch 11, worin die Bestandteile einen Füllstoff ausgewählt aus Zuckern, Zuckeralkoholen, Cellulose und Calciumphosphaten und -sulfaten umfassen.

13. Verfahren gemäss irgendeinem der Ansprüche 1 bis 10 für die Herstellung von Tabletten umfassend Nahrungsmittel oder Chemikalien zur Auflösung in Wasser oder wässrigen Lösungsmitteln.

## Revendications

1. Procédé de fabrication de comprimés à désintégration rapide, **caractérisé en ce que** les composants sous forme pulvérisée sont mis en contact avec un gaz ou un mélange de gaz liquéfié sous pression, homogénéisés, introduits dans des moules sous une pression comprise entre 101 325 Pa (pression normale ou 1,01325 bar) et 10⁷ Pa (100 bar) et décomprimés.

2. Procédé selon la revendication 1, dans lequel le gaz liquéfié sous pression a un point d'ébullition inférieur à 0°C à 101 325 Pa (pression normale).

3. Procédé selon la revendication 1 ou 2, dans lequel le gaz liquéfié sous pression est choisi parmi les fluoroalcanes, les fluorochloroalcanes, les alcanes inférieurs et les éthers de point d'ébullition bas, ainsi que leurs mélanges.

4. Procédé selon la revendication 3, dans lequel le mélange de gaz est un mélange azéotropique.

5. Procédé selon l'une quelconque des revendications 1 à 4, dans lequel le gaz liquéfié sous pression est choisi parmi TG 227 (1,1,1,2,3,3,3-heptafluoropropane), TG 134a (1,1,1,2-tétrafluoroéthane), le propane, le n-butane, l'isobutane et l'éther diméthylique, et leurs mélanges.

6. Procédé selon l'une quelconque des revendications 1 à 5, dans lequel le gaz ou mélange de gaz liquéfié sous pression comprend également un solvant de point d'ébullition bas ayant un point d'ébullition compris entre 20 et 100°C à 101 325 Pa (pression normale).

7. Procédé selon la revendication 6, dans lequel le gaz ou mélange de gaz liquéfié et le solvant supplémentaire forment un mélange azéotropique.

8. Procédé selon l'une quelconque des revendications 1 à 7, dans lequel le gaz liquéfié sous pression comprend également du dioxyde de carbone.

9. Procédé selon l'une quelconque des revendications 1 à 8, dans lequel le gaz ou mélange de gaz liquéfié sous pression et l'éventuel solvant de point d'ébullition bas sont tout d'abord utilisés pour dissoudre un agent de liaison, et la solution résultante ajoutée aux autres composants solides sous forme pulvérisée sous pression.

10. Procédé selon l'une quelconque des revendications 1 à 9, dans lequel les composants comprennent un agent de liaison choisi parmi le phtalate et le succinate d'hydroxypropylcellulose, l'éthylcellulose, le phtalate de cellulose, le phtalate de polyvinyle et les copolymères d'acrylate d'acide méthacrylique.

11. Procédé selon l'une quelconque des revendications 1 à 10 pour la fabrication de comprimés pharmaceutiques pour un usage oral.

12. Procédé selon la revendication 11, dans lequel les composants comprennent une charge choisie parmi les sucres, les polyols, la cellulose et les phosphates et sulfates de calcium.

13. Procédé selon l'une quelconque des revendications 1 à 10 pour la fabrication de comprimés comprenant des produits alimentaires ou chimiques pour une désintégration dans de l'eau ou des solvants aqueux.
